# EUROPEAN PATENT APPLICATION

(11) **EP 2 422 769 A1**
(43) Date of publication of application: **29.02.2012**
(21) Application number: 10008555.4
(22) Date of filing: 17.08.2010
(51) Int. Cl.: A61K 9/127, A61K 31/137, A01N 1/02, A61P 37/06

(54) **Compositions and methods for improved organ transplant preservation and acceptance**

(71) Applicant: Novaliq GmbH, 69120 Heidelberg (DE)
(72) Inventor: Günther, Bernhard, 69221 Dossenheim (DE); Theisinger, Sonja, Dr., 68229 Mannheim (DE); Theisinger, Bastian, Dr., 68229 Mannheim (DE)
(74) Representative: Cremer, Karsten

(57) **Abstract**

The invention provides a novel aqueous composition for the storage and preservation of transplants, such as organ or tissue allografts. The composition comprises the compound N-octanoyl dopamine in solubilised form. The composition may also be administered as a pre-treatment of transplant donors. Moreover, it may be used in transplant recipients, optionally in combination with immunosuppressants.

## Description

### BACKGROUND

The present invention relates to the transplantation of organs and tissues. More specifically, it relates to the pre-treatment of transplant donors, the preservation of transplants after their withdrawal from donors, and the treatment of transplant recipients in order to maximise their benefit from the transplant

Transplantation means the removal of an organ or tissue from one body and its implantation into another, for the purpose of replacing a severely damaged or absent organ or tissue. For the recipient of a major organ transplant such as a heart, lung, kidney, liver or pancreas, transplantation is often the only realistic chance for mid- and long-term survival in view of the severity of the underlying disease or injury.

Today, it is estimated that at least several ten thousand major transplantations are performed every year. The most frequently transplanted organ is the kidney, of which about 25,000 are transplanted per year in the USA alone, followed by the liver, heart, lung, and pancreas. The success of these life-extending procedures has markedly increased over the past decades. For example, the current five-year survival rate for heart transplant recipients is about 73 % for males and 67 % for females. In the case of kidneys, the typical patient will live ten to fifteen years longer with a transplant than if kept on dialysis. For liver transplantation, the recipients have a 58 % chance of surviving 15 years.

At the same time, there is still a large number of transplantations which fail. The causes of transplant failure are often associated with (a) pre-existing diseases of the donor or pre-transplantation injury of the organ, (b) damage to the transplant after withdrawal, e.g. during transport and storage, and/or (c) transplant rejection by the immune system of the recipient

Brain death is considered one of the major causes of pre-transplantation injury of allografts. According to the damage hypothesis, pre-transplantation injury has substantial impact on transplantation outcome. Especially in kidney transplantation, grafts retrieved from brain dead donors indeed show a decreased long-term survival compared to those from living donors. Graft injury in brain dead donors probably occurs via several pathways, one of them relating to haemodynamic events, another to inflammatory responses of organs including the kidneys.

Recently, it has been shown that a pre-treatment of donors with low-dose dopamine before kidney withdrawal has a beneficial effect on the graft function (Schnuelle P et al., Effects of donor pre-treatment with dopamine on graft function after kidney transplantation: a randomized controlled trial. JAMA. 2009 Sep 9;302(10):1067-75). However, the altered catecholamine clearance in brain-dead donors makes the dosing of dopamine difficult and can lead to hypertension and tachycardia.

Moreover, it has been proposed that chemically modified dopamines, in particular N-octanoyl dopamine (NOD), may be even better suitable for donor pre-treatment than dopamine itself, and may also be used advantageously in organ preservation solutions (Lösel RM et al)., N-octanoyl dopamine, a non-haemodynamic dopamine derivative, for cell protection during hypothermic organ preservation. PLoS One. 2010 Mar 16;5(3):e9713; see also WO 2009/015752 A2). An advantage of these derivatives is that they exhibit strong reducing capacity, but at the same time they are largely devoid of haemodynamic activity and, by means of their increased lipophilicity over dopamine, have a potential for substantially increased cellular uptake.

However, it is difficult to deliver lipophilic dopamine derivatives effectively. They are chemically unstable, prone to oxidation, and poorly soluble in aqueous media. As suspensions, they cannot be injected or infused intravenously and, when added to organ preservation solutions, they will precipitate and lead to unreliable effectiveness.

The preservation of organs and tissues during transport and storage is typically achieved by keeping them in a preservation solution and cooling them to just above freezing point. A common preservation solution is based on histidine-tryptophan-ketoglutarate (HTK), aiming at the inactivation of organ function by withdrawal of extracellular sodium and calcium, together with intensive buffering of the extracellular space by means of histidine/histidine hydrochloride, so as to prolong the period during which the organs will tolerate interruption of oxygenated blood. Alternative solutions are the Euro-Collins (EC) and the University of Wisconsin (UW) solution. The latter mimics the properties of intracellular fluids, but also comprises a polymer (hydroxyethyl starch) to prevent oedema, and additives for scavenging free radicals. As mentioned, it has also been proposed to add dopamine or lipophilic dopamine derivatives to such organ perfusion and preservation mixtures in order to reduce cold preservation injury.

Transplant rejection is another major cause of graft failure. In principle, the immune system of the recipient attacks the graft as foreign material within the body and attempt to destroy it Transplant rejection can be somewhat reduced in severity and incidence by carefully serotyping donors and recipients in order to determine the most appropriate matches. Nevertheless, some episode of rejection occur in many if not most transplant recipients, and the severity of the immune response can only be managed with the use of immunosuppressant drugs.

The immune reactions of recipients may be classified as hyperacute, acute, and chronic rejection. Hyperacute rejection is a complement-mediated response in recipients with pre-existing antibodies to the donor. Such reactions may occur within minutes and a transplant must be immediately removed to prevent a severe systemic inflammatory response and extensive agglutination of the blood. The risk of hyperacute rejection is especially associated with kidney transplants.

Acute rejection involves the infiltration of T-cells and other immune cells in the transplant The T-cells, via various mechanisms, cause cells in the transplanted tissue to lyse, or produce cytokines which lead to tissue necrosis. Since the T-celis must first differentiate before this type of reaction occurs, acute rejection typically begins only after a latency of one or more weeks after transplantation. The highest risk for acute rejection is in the first three months. The severity of acute rejection is not always dramatic; some degree of rejection may be handled by appropriate immunosuppressive medication.

Recurrent and poorly controlled episodes of acute rejection may eventually lead to the manifestation of a chronic transplant rejection, which summarises most forms of chronic inflammatory and immune response against the transplanted tissue. A related condition is chronic allograft vasculopathy, which describes the long-term loss of organ function associated with fibrosis of the vasculature of the transplanted tissue. Chronic transplant rejection is irreversible and cannot be treated effectively.

Immunosuppressive drugs effective to control acute rejection reactions include corticosteroids, calcineurin inhibitors, mTOR inhibitors, anti-proliferative agents, and antibodies against T-cells or B-cells, some of which target specific surface proteins thereon (e.g. CD20, CD25). Particularly important in the prevention and management of transplant rejections are the macrolide immunosuppressant acting as calcineurin inhibitors, e.g. ciclosporin A and tacrolimus, or mTOR inhibitors, such as sirolimus and everolimus. In fact, substantial therapeutic benefit for transplant recipients was brought about by the advent of the first macrolides, in particular ciclosporin, and later tacrolimus. Later, compounds with related structure and activity include pimecrolimus, everolimus, sirolimus, deforolimus, everolimus, temsirolimus, and zotarolimus.

Most, if not all of the macrolides used for immunosuppression are poorly soluble compounds with problematic bioavailability. Their use is associated with substantial adverse effects. Ciclosporin A, for example, exhibits significant nephrotoxicity and hepatotoxicity, but may also lead to gingival hyperplasia, convulsions, peptic ulcers, pancreatitis, fever, vomiting, diarrhoea, confusion, hypercholesterolemia, dyspnoea, numbness and tingling particularly of the lips, pruritus, high blood pressure, and an - like all immunosuppressants - increased vulnerability to opportunistic fungal and viral infections. Tacrolimus may induce cardiac damage, hypertension, blurred vision, liver and kidney problems seizures, tremors, hyperkalemia, hypomagnesaemia, hyperglycaemia, diabetes mellitus, itching, insomnia, and neurological problems such as posterior reversible encephalopathy syndrome confusion, loss of appetite, weakness, depression, cramps, and neuropathy.

In addition, there is some evidence that immunosuppressants such as ciclosporin, tacrolimus and the like are associated with an increased risk of malignancies, e.g. non-Hodgkin's lymphoma and melanoma, in transplant recipients. The risk appears to be related to the dosing and duration of treatment

In order to further increase the physiological function of allografts and reduce the risk of transplant failure, there is a need for improvements in donor pre-treatment for minimising cold ischaemia damage. Moreover, there is a need for an improved preservation of transplants until implantation in order to further reduce the risk of cold preservation injury. Finally, there is also a need for a more effective and better tolerated pharmacotherapy of transplant recipients in order to maximise their benefit from the transplant and minimise the risk of severe rejection reactions.

These needs are addressed by the present invention, whose object it is to provide improvements in donor pre-treatment, allograft preservation, and recipient treatment, which improvements overcome one or more disadvantages of prior art methods and compositions. Still further objects will be understood in the light of the description and the patent claims.

### SUMMARY OF THE INVENTION

The present invention provides a pharmaceutical composition comprising an effective amount of N-octanoyl dopamine, a physiologically acceptable aqueous solvent, and a physiologically acceptable amphiphilic excipient In the composition, N-octanoyl dopamine is present in a molecularly or colloidally dispersed state.

In the composition of the invention, N-octanoyl dopamine is incorporated in solubilised form, For example, it may be formulated as a micellar solution, as a colloidal liposome dispersion, or as a microemulsion. In the case of a micellar solution, it is preferably solubilised by a nonionic surfactant such as a polysorbate.

At the same time, the composition is formulated to protect N-octanoyl dopamine from degradation. While the compound is poorly compatible with a number of solubilising excipients, it has been found by the inventors that certain nonionic surfactants, but also certain vesicle-forming amphiphilic lipids, may in fact be used to formulate N-octanoyl dopamine into a solubilised and stable aqueous formulation.

The composition is preferably adapted for parenteral administration. It may be administered systemically or locally to a transplant donor for pre-treatment and prevention of ischaemic damage.

According to a further embodiment, it is used *in vitro* for the preservation of allografts in order to minimise cold preservation injury. For this purpose, it may, for example, be added to a conventional organ preservation medium.

In a yet further embodiment, N-octanoyl dopamine is administered to an allograft recipient, e.g. by intravenous injection. Typically, the recipient is a patient co-treated with at least one immunosuppressant drug, such as a calcineurin inhibitor, e.g. ciclosporin.

### BRIEF DESCRIPTION OF THE DRAWINGS

Fig.1 shows the mitigation of ATP loss in cardiomyocytes after 8 hours of cold storage as effected by the composition of Example 1 in comparison with untreated and conventionally (dopamine) treated cells.
Fig. 2 shows the inhibition of LDH-release after 8 hours of cold storage in the presence of the composition of Example 1, in comparison with untreated and conventionally (dopamine) treated cells.

### DETAILED DESCRIPTION OF THE INVENTION

According to the present invention, a pharmaceutical composition is provided which comprises an effective amount of N-octanoyl dopamine, a physiologically acceptable aqueous solvent, and a physiologically acceptable amphiphilic excipient In the composition, N-octanoyl dopamine is present in a molecularly or colloidally dispersed state.

It has been found by the inventors that such composition in which N-octanoyl dopamine is present in solubilised form provides a means for using the compound more effectively than hitherto known. Surprisingly, the solubilised formulations of the invention not only allow the compound to be administered by various parenteral routes including intravenous injection or infusion, but also increase its protective activity. At the same time, the composition simultaneously overcomes the challenges resulting from properties of N-octanoyl dopamine which make it difficult to formulate and administer the compound, in particular its poor water solubility and its pronounced chemical instability.

N-octanoyl dopamine, also referred to as N-octanoyl-4-(2-aminoethyl)benzene-1,2-diol or N-octanoyl-4-2-(3,4-dihydroxyphenyl)ethylamine, is a lipophilic acyl derivative of the catecholamine neurotransmitter, dopamine. As used herein, the term N-octanoyl dopamine also includes any salts, isomers, and solvates of N-octanoyl dopamine.

An effective amount means an amount appropriate for achieving the effect within the context of the intended use. The effective amounts of N-octanoyl dopamine may, for example, differ between organ preservation solutions and donor pre-treatment injections.

A physiologically acceptable aqueous solvent is water or an aqueous solution of compounds, in particular pharmaceutical excipients, which are considered safe with respect to the incorporated amount and the intended use. For example, the aqueous solvent may be sterile isotonic sodium chloride solution, or a sterile buffer solution.

Similarly, a physiologically acceptable amphiphilic excipient is an amphiphilic compound, in particular a surfactant, which may be used as a pharmaceutical excipient in that it is safe and well-tolerated at least at the incorporated level and in view of the intended use, taking into consideration the route and frequency of administration.

The molecularly dispersed state refers to a true molecular solution. In a liquid solution, the molecules of the solute(s) are individually solvated and surrounded by solvent molecules. In contrast, the colloidally dispersed state means that a material, in this case N-octanoyl dopamine, is present in structures having a colloidal size, i.e. they are substantially larger than the respective molecules but too small to be visible to the unaided eye. Colloids typically have a diameter of between approximately 1 and 500 nanometres. (H. Stricker, Physikalische Pharmazie, 3rd Edition, page 440). Therefore, colloidal structures are practically not visible with a light microscope and do not result in market turbidity of the solution, but rather in opalescence.

Colloidal structures of various types are known to exist in different types of colloidal liquids. In isotropic colloidal solutions, the properties of the solution are the same regardless of the direction of measurement. In other words, in the isotropic state, all directions are indistinguishable from each other. For example, a micellar solution may be isotropic. In anisotropic colloidal solutions, there is orientation and/or alignment of molecules which causes the physical properties of the solution to vary for different directions of measurement. Such anisotropic solutions are referred to as liquid crystals, or liquid-crystalline phases, or mesophases.

In one of the preferred embodiments, the composition of the invention comprises N-octanoyl dopamine in this colloidally dispersed state. The colloidal particles that are dispersed in the aqueous solvent will typically contain both the amphiphilic excipient and the active ingredient As used herein, colloidal dispersion may also be referred to as a colloidal solution.

According to the further preferred embodiment, the composition comprises an amphiphilic excipient selected from the group of nonionic surfactants. Pharmaceutically acceptable nonionic surfactants include, for example, tyloxapol, poloxamers such as poloxamer 188, poloxamer 407, Pluronic F68LF or Lutrol F68, Pluronic F127, Pluronic L-G2LF and Pluronic L62D, polysorbates such as polysorbate 20, polysorbate 60, and polysorbate 80, polyoxyethylene castor oil derivatives, sorbitan esters, polyoxyl stearates, and mixtures of two or more thereof. In a specific embodiment, the nonionic surfactant is a polysorbate. In a further specific embodiment, the nonionic surfactant is polysorbate 80.

Optionally, the composition comprises two or more amphiphilic excipients or surfactants. In one of the preferred embodiments, two or more nonionic surfactants are used in combination. For example, a polysorbate, such as polysorbate 20 or 80, may be combined with Cremophor EL or Cremophor RH.

The amphiphilic excipient or surfactant, or combination of surfactants, may be incorporated in such an amount that it forms micelles in which the N-octanoyl dopamine is solubilised. Generally speaking, micelles are colloidal aggregates of amphiphilic molecules in a solvent. They may be spherical, but can also have very different shapes. In an aqueous system, a typical spherical micelle comprises surfactant molecules whose hydrophilic moieties are in contact with the surrounding solvent, sequestering the hydrophobic molecular regions in the micelle centre. Poorly water-soluble lipophilic compounds may be dissolved in the core of such micelles.

In order for micelle formation to occur, the concentration of the surfactant (or surfactants) must be above the critical micelle concentration (CMC). Therefore, the amount of surfactant in the composition should be selected above this concentration if a micellar solution is aimed at. Moreover, the amount of surfactant should be selected sufficiently high as to solubilise the incorporated amount of N-octanoyl dopamine. At the same time, the amount of surfactant must be low enough to avoid undesirable effects in the donor of a transplant, the transplant itself, or the recipient of the transplant

In one particular embodiment, the amount of amphiphilic excipient is at least about 0.05 wt.-%. According to a further embodiment, the amount is from about 0.1 wt.-% to about 30 wt.-%, or from about 0.5 wt.-% to about 20 wt.-%, respectively. For example, it has been found that already at a level of 0.5 wt.-%, polysorbate 20 and polysorbate 80 are individually capable of solubilising substantial amounts of N-octanoyl dopamine, e.g. 40 µmol/mL. However, if the composition is to be used as a concentrate to be added to a commercial solution for tissue and organ preservation, the concentrate itself may also comprise the amphiphilic excipient at a relatively high concentration, taking into account the dilution factor. In a further embodiment, the ratio of N-octanoyl dopamine to the amphiphilic excipient is selected in the range from about 5:1 to about 1:50.

In a further preferred embodiment, the composition is free of organic solvents or co-solvents such as ethanol, glycerol, propylene glycol, or polyethylene glycol. According to another preferred embodiment, the composition may contain small amounts of such solvents or co-solvents, such as up to about 2 wt.-%.

The composition may comprise further Inactive pharmaceutical ingredients as required or appropriate. For example, it may comprise one or more excipients for adjusting the tonicity of the formulation. It is preferred that the composition is adapted to exhibit an osmotic pressure of roughly 310 mOsmol/kg, such as in the range from about 200 to about 450 mOsmol/kg, or in the range from about 250 to about 400 mOsmoI/kg, or in the range from about 280 to about 350 mOsmol/kg, respectively. If the intended use is the preservation of allografts during storage and transport, it should be ensured that, optionally after dilution with a conventional organ preservation solution, the composition has a physiological osmolality of about 300 to 330 mOsmol/kg. Suitable excipients for adjusting the osmotic pressure include, for example, salts, sugars, sugar alcohols, and amino acids. Among the salts, buffer salt or sodium chloride are particularly suitable. Useful sugars and sugar alcohols include, for example, glucose, raffinose, trehalose, sorbitol, and mannitol, to mention only a few.

Moreover, the composition may comprise one or more excipients for adjusting the pH value, which is preferably selected in the range from about pH 3 to about pH 8. More preferably, the pH is not higher than about 7, such as from about pH 4 to about pH 7.0, or from about pH 4.5 to about pH 6.5. If the intended use is the preservation of allografts during storage and transport, it should be ensured that, optionally after dilution with a conventional organ preservation solution, the composition exhibits a pH of about 7.0 to 7.5. Suitable excipients for adjusting the pH include physiologically acceptable organic or inorganic acids, bases, and buffer salts. The latter salts may at the same time function as physiological electrolytes, such as salts of sodium, potassium, magnesium, and calcium.

The composition may further comprise one or more stabilisers, such as complexing or chelating agents like EDTA, and/or antioxidants such as vitamin E or vitamin E derivatives, ascorbic acid, sulphites, hydrogen sulphites, gallic acid esters, butyl hydroxyanisole, butyl hydroxytoluene or acetylcysteine; viscosity-increasing agents such as water-soluble polymers; preservatives (in case the composition is to be packaged in multiple-dose containers and used for parenteral administration); lactobionic acid, allopurinol, glutathione, adenosine; amino acids such as histidine, tryptophan, glutamic acid, aminoglutamic acid, or ketoglutarate.

Optionally, the invention may be carried out by formulating a powder or liquid concentrate from which a composition as described herein can be reconstituted. For example, for achieving an extended shelf life it may be useful to formulate the solid components of the composition as a sterile lyophilised powder which may, prior to its use, be dissolved or dispersed in an appropriate aqueous carrier or diluent Alternatively, a liquid concentrate may be formulated which, upon dilution with an aqueous medium, yields the final composition to be used for transplant donor pre-treatment, allograft preservation, or treatment of transplant recipients. Such liquid concentrate not only has the advantage of having a low weight and volume which makes it easier to manufacture, transport, store, and handle it, but also provides an opportunity to depart from physiological parameters such as pH or osmolality during storage, e.g. with an eye on an extended shelf life. The physiological properties required for its use are then achieved by appropriately diluting the concentrate.

In another embodiment, the composition is in the form of a microemulsion. As used herein, a microemulsion is a clear, thermodynamically stable, optically isotropic mixture of a lipophilic component, a hydrophilic component, and an amphiphilic components. Typically, a microemulsion forms spontaneously when the components are combined and mixed with each other, without requiring high energy input as is normally required for the formation of an "ordinary" emulsion. Microemulsions may have a colloidal lipophilic phase dispersed in a hydrophilic phase, or a hydrophilic phase colloidally dispersed in a lipophilic phase. The size of the dispersed phases is usually in the range from about 5 nm to about 400 nm, and most often below about 200 nm. In one of the preferred embodiments of the invention, the particle size is from about 5 nm to about 100 nm. In terms of its rheological properties, the microemulsion may be in the form of a liquid or a gel, i.e. in liquid or semisolid form. In a preferred embodiment, the microemulsion is in liquid form. If a microemulsion is used, the lipophilic component is preferably selected from excipients which are per se suitable for parenteral use. For example, a highly purified triglyceride oil or semisynthetic medium-chain triglycerides may be used.

In a further embodiment, the amphiphilic excipient Is a vesicle-forming phospholipid. In this case, the composition is designed as a colloidal dispersion of liposomes, wherein the liposomes incorporate the N-octanoyl dopamine. As used herein, a liposome is a vesicle formed from at least one bilayer, wherein the bilayer is composed of aggregated (or assembled) amphiphilic lipids. The bilayer exhibit some similarity with biological membranes in that it is hydrophilic towards the inside and outside of the vesicle, whereas the lipophilic region is sandwiched in between these hydrophilic regions. Larger liposomes often have two or more concentric bilayers. Small liposomes tend to be rather spherical, but larger vesicles may exist in various shapes.

Depending on the selected preparation method and manufacturing conditions, the resulting liposomes may be described as multilamellar vesicles (MLV), small unilamellar vesicles (SUV), or large unilamellar vesicles (LUV). MLVs differ from SUVs and LUVs in that MLVs have two or more lipid bilayers. Hence, ldl,Vs appear useful in particular for being loaded with lipophilic drug substances which dissolve in, or associate with, the lipophilic regions of the vesicle membranes. In contrast, SWs and LUVs are especially useful for the encapsulation of hydrophilic compounds within the aqueous compartment of the liposomes. Typically, MLVs have a diameter from about 200 nm up to several microns. SUVs typically range from about 80 nm to about 200-300 nm, whereas LUVs are normally understood to be larger than about 200-300 nm in average. Within the context of the invention, the diameters are understood as z-averages as measured with laser diffraction or photon correlation spectroscopy. In the context of the present invention, colloidal liposomes should be used, and very large MLVs may not fall into this category.

The amphiphilic lipids from which the liposomes are composed typically include at least one phospholipid. Phospholipids are amphiphilic lipids comprising a phosphate group, which is negatively charged and thus substantially hydrophilic. Phospholipids may be classified as glycerophospholipids (or phosphoglycerides, characterised by the presence of a glyceryl moiety) or phosphosphingolipids (or ceramides, such as sphingomyelin). Liposomes may contain native, semisynthetic and/or synthetic phospholipids.

Typically, liposomes comprise at least one glycerophospholipid (or phosphoglyceride). Such glycerophospholipids are in fact the most commonly used vesicle-forming lipids in liposomes. Commonly used glycerophospholipids include those which are derived from native lecithins, such as soy or egg lecithin, or from the (partial) hydration products thereof. Lecithins contain high amounts of phosphatidylcholines, but may also comprise smaller amounts of phosphoric acid, choline, fatty acids, glycerol, glycolipids, triglycerides, phosphatidylethanolamines, and phosphatidylinositol. Phosphatidylcholines are glycerophospholipid that comprise choline as a head group, in contrast to phosphatidylethanolamines and phosphatidylglycerols.

In phosphatidylcholines, two hydroxyl group of the glyceryl residue are linked via ester bonds to acyl groups, which are typically derived from medium to long chain fatty acids. Common acyl groups in phosphatidylcholines (but also in phosphatidylethanolamines and phosphatidylglycerols) used as constituents of liposomes include myristoyl, palmitoyl, stearoyl, and oleoyl groups.

Due to the negative charge of the phosphate group and the positive charge of the choline, phosphatidylcholines are always zwitterionic (sometimes also referred to as neutral). Phosphatidylethanolamines are also zwitterionic over large pH-ranges, but can exist as anions in basic environments. Phosphatidylglycerols are anionic.

Besides one or more glycerophospholipids, liposomes may comprise one or more lipids which are themselves not capable of forming bilayers, but which modify or stabilise such bilayers. An example of such membrane-modifying lipid is cholesterol.

Methods for the preparations and characterization of liposomes and liposome preparations are known as such to the skilled person. Often, multilamellar vesicles will form spontaneously when amphiphilic lipids are hydrated, whereas the formation of small unilamellar vesicles usually requires a process involving substantial energy input, such as ultrasonication or high pressure homogenization. Further methods for preparing and characterizing liposomes have been, for example, described by S. Vemuri et al. [Preparation and characterization of liposomes as therapeutic delivery systems: a review. Pharm Acta Helv. 1995, 70(2):95-111].

Of the known liposomes, those which may be used according to the invention have a predominantly colloidal size, i.e., their average particle size lies below about 500 nm. Also preferred is a diameter of up to about 300 nm, or not higher than 200 nm, respectively. Such average particle size will usually allow sterile filtration through a filter with a pore size of 0.22 µm, which is a significant advantage in case the composition is not stable enough to withstand heat sterilization.

The composition of the invention may be used as a medicine or as a liquid medium for the preservation and storage of organ or tissue allografts. When used for allograft preservation, it may be injected or infused - optionally after reconstitution or dilution with a conventional organ storage solution - directly into the vascular system of an organ before and/or immediately after its removal from a donors. Thus the organ vasculature is flushed with the composition, which is subsequently left within the vasculature for storage and transport. After implantation and before perfusion is established in the recipient, the transplant should be flushed free of the preservation and storage solution using a physiological plasma volume expander or the like.

Alternatively, the composition may be injected or infused systemically, e.g. intravenously, to the donor. According to this use, the donor is pre-treated with N-octanoyl dopamine, which is particularly useful in that the protective function of the compound can be initiated much earlier than just at the time of allograft removal. Moreover, in the case of multi-organ removal e.g. from a brain-dead donor, this regimen allow the simultaneous onset of protection for all organs of interest

In a further embodiment, the composition is used as a medicine for administration to the transplant recipient, in particular for the purpose of prevention and treatment of transplant rejection.

Surprisingly, the inventors have found that N-octanoyl dopamine is capable of inhibiting T-cell proliferation at therapeutically useful concentrations. Inhibition appears to be downstream of early T-cell receptor signalling events, and is associated with the inhibition of NFκB (nuclear factor kappa-light-chain-enhancer of activated B cells) and AP-1 (activator protein 1). The effects were found to be dependent on the redox activity, i.e. N-octanoyl dopamine loses its T-cell inhibitory capability as it becomes oxidised.

Moreover, it was also unexpectedly found that N-octanoyl dopamine and calcineurin inhibitors such as ciclosporin A act synergistically in their inhibition of T-cells, so that the concentration of a calcineurin inhibitor required for substantial T-cell inhibition is much lower in the presence of N-octanoyl dopamine than in its absence. Based on this finding, the present invention teaches, inter alia, that transplant recipients who receive a calcineurin inhibitor such as ciclosporin A may advantageously be co-treated with N-octanoyl dopamine. The concurrent administration of N-octanoyl dopamine allows for a considerable reduction of the dose of the calcineurin inhibitor, which will bring about the benefit of a substantial reduction in the occurrence and severity of dose-dependent adverse reactions of the calcineurin inhibitor. In the case of ciclosporin A and tacrolimus, for example, the invention provides a means for reducing in particular their nephrotoxic potential which has been one of the major disadvantages of their therapeutic use in transplant recipients.

For the treatment of transplant recipients, N-octanoyi dopamine, optionally in the form of the composition as described herein-above, may be administered by any route which will lead to the systemic availability of N-octanoyl dopamine, or to its local bioavailability in the transplant. In particular, N-octanoyl dopamine or the composition of the invention may be administered parenterally or orally. As used herein, parenteral administration refers to any invasive type of administration by injection or infusion, including intravenous, intraarterial, subcutaneous, intramuscular, locoregional, intraluminal, and intradermal administration. In a preferred embodiment, the route is selected from intravenous, intraarterial, subcutaneous, and intramuscular administration.

For oral administration, N-octanoyl dopamine may, for example, be formulated as a tablet, hard capsule, or softgel, using common pharmaceutical excipients as known to the person skilled in the art.

Further embodiments will become obvious from the following examples which illustrate the invention in some of its major aspects, without limiting the scope thereof.

### EXAMPLES

### Example 1

A, solubilised formulation of N-octanoyl dopamine was formulated with polysorbate 80 (Tween 80) as amphiphilic excipient In short, N-octanoyl dopamine and polysorbate 80 were added to isotonic (0.9 wt.-%) sodium chloride solution such as to obtain an N-octanoyl dopamine concentration of 1.116 mg/mL (4 µmol/ml) and a polysorbate concentration of 5 mg/mL. The pH was adjusted to pH 6.5. The mixture was filled into vials, closed with a rubber stopper and aluminium cap, and autoclaved for 10 minutes at 121 °C and subsequently agitated during cooling. A colourless, clear or slightly opalescent solution was obtained. The solution was physically and chemically stable at room temperature for at least 4 weeks.

### Example 2

In analogy to example 1, a solubilised formulation of N-octanoyl dopamine was formulated with poloxamer 407 (Pluronic 127) as amphiphilic excipient The same amounts and procedures were used. Again a stable and only slightly opalescent liquid was obtained.

### Example 3

Example 1 was repeated, except that the amount of N-octanoyl dopamine and of the polysorbate were increased by the factor of 10 (to 11.16 mg/mL and 50 mg/mL, respectively). Again, a colourless, clear or slightly opalescent and stable solution was obtained, demonstrating that surprisingly small concentrations of the polysorbate are capable of solubilising therapeutically relevant amounts of N-octanoyl dopamine.

### Example 4

Examples 1 and 3 were repeated, except that acetylcysteine was used as an additional excipients at a concentration of 0.189 mg/mL. The pH of the mixtures was 4.5. The resulting liquid was colourless and only slightly opalescent. Under stress conditions, it remained colourless for a still longer time than the formulations of examples.1 and 3.

### Example 5

Two samples of the formulation of example 4 having a N-octanoyl dopamine concentration of 11.16 mg/mL were individually mixed with either University of Wisconsin solution (Viaspan, or UW) or histidine-tryptophan-ketoglutarate (HTK) solution (Custodiol) at a volume ratio of 1: 4 (1 part of the inventive composition plus 4 parts of the conventional solution). After 117 hours at 4 °C, the mixtures were still clear and showed no signs of oxidation of N-octanoyl dopamine, which usually occurs very rapidly when mixed with UW or HTK, leading to a brownish-pink colour.

### Example 6

N-octanoyl dopamine (11.16 mg/mL) and soy bean lecithin (Lipoid 575) (50 mg/mL) were added to an aqueous solution of glucose (5 wt.%). The pH was adjusted to pH 6.5. The mixture was ultrasonicated for 4 minutes and subsequently autoclaved for 10 minutes at 121 °C. Upon cooling, an almost clear, only slightly opalescent liquid was obtained. Its colour was slightly yellow due to the lecithin.

### Example 7

Example 6 was repeated, except that acetylcysteine was used as an additional excipient at a concentration of 0.189 mg/mL. The pH of the mixtures was 4.5. The resulting liquid was physically and chemically stable.

### Example 8

N-octanoyl dopamine (223.2 mg), polysorbate 80 (1,000 mg) and ethanol (50 mg) were weighed, mixed and heated to 121°C for 10 minutes. Subsequently, the mixture was cooled to room temperature under agitation. The resulting concentrate was diluted with water for injection or sterile isotonic sodium chloride solution in various ratios, always yielding clear and substantially colourless solutions.

### Example 9

The formulation obtained according to example 1 was tested as to whether it is capable of inhibiting TNF-α-mediated inflammation. Human umbilical vein endothelial cells (HUVEC) were stimulated with TNF-α (tumour-necrosis-factor alpha) in the absence or presence of various concentrations of the N-octanoyl dopamine formulation. Affymetrix Gene Expression Profiling, quantitative PCR, Western-blotting and NF-_{K}B activation were performed to assess the anti-inflammatory potential of the composition of the invention. As a comparator, a surfactant-free aqueous dispersion of N-octanoyl dopamine, which is not an embodiment of the invention, was also tested.

In result, gene expression profiling revealed that a wide range of pro-inflammatory genes were down-regulated by the composition, amongst these adhesion molecules and chemokines. Although HO-1 (haeme oxygenase 1) was strongly upregulated by N-octanoyl dopamine, HO-1 expression did not contribute to the anti-inflammatory effect. While HO-1 was already significantly induced at 1 µM of N-octanoyl dopamine, inhibition of VCAM-1 (vascular cell adhesion molecules-1) expression required 50 µM of the compound. Apart from HO-1, genes belonging to the Ubiquitin Proteasome System (UPS), e.g. de-ubiquitinilating enzymes E3-ligases and proteasome sub-units, were significantly upregulated by the composition. It did not inhibit degradation of IκBα (nuclear factor of kappa light polypeptide gene enhancer in B-cells inhibitor, alpha), but was able to inhibit sustained NFκB activation. The comparator formulation was much less effective, i.e. required substantially higher concentrations for achieving the same effects.

It may be concluded from the results that the composition of the invention showed potent anti-inflammatory effects. Since pre-transplantation injury severely affects transplantation outcome, the composition will be useful for donor preconditioning to reduce brain-death-induced inflammation and to maintain organ quality especially after prolonged cold preservation.

### Example 10

The formulation obtained according to example 3 was tested as to whether it exhibits anti-inflammatory effects *in vivo*. Acute renal failure (ARF) rats were pre-treated with an intravenous bolus of the composition which was equivalent to 0.67 mg of N-octanoyl dopamine. The kidneys were harvested after 1 or 5 days. Renal function was measured. Renal inflammation was assessed by immunohistochemistry and EMSA, (electrophoretic mobility shift assay).

In result, the composition of the invention significantly improved renal function compared to saline controls. Immunohistochemistry revealed a reduced number of monocytes in the treated rats compared to controls. Furthermore NFkB was down-regulated in renal tissue treated with the composition. Thus it was demonstrated that the composition has potent anti-inflammatory effects *in vivo.* Its administration not only mitigates deterioration in renal function but also reduces renal inflammation in the setting of ischemia reperfusion.

### Example 11.

The formulation obtained according to example 1 was tested as to whether it is capable of influencing T-cell proliferation. A cell-culture system was used in which freshly isolated T-cells were polyclonally activated by anti-CD3/anti-CD28 beads. The cells were allowed to proliferate for at least 10 days in the presence or absence of different dilutions of the composition of the invention. T-cell proliferation was assessed on day 3, 5, 7 and 10 by means of 3H-thymidine incorporation. In this model, T-cell proliferation is maximal at day 3 and gradually declines at day 10. In the presence of the composition there was a dose-dependent inhibition in T-cell proliferation, which was reversible depending on the concentration used. For example, a concentration of 25 µM N-octanoyl dopamine was without effect, 50 µM inhibited T-cell proliferation at day 3 (hereafter the T-cells started to proliferate), and at 100 µM, T-cell proliferation was inhibited at day 3, day 5 and partly at day 7. The inhibitory effect was found to be dependent on the redox activity, since inhibition was abrogated by oxidation of N-octanoyl dopamine and was not observed with structurally related compounds that lacks redox activity.

The experiments were repeated, except that freshly isolated T-cells were stimulated with anti-CD3/antio-CD28 antibodies in the presence of the composition diluted to a concentration of 50 µM of N-octanoyl dopamine and various concentrations of ciclosporin A or tacrolimus. Similar concentrations of ciclosporin A or tacrolimus were also tested in the absence of the composition.

In result, both ciclosporin A and tacrolimus dose-dependently inhibited T-cell proliferation, with a maximal inhibition at 10 µM for ciclosporin A and 1 µM for tacrolimus. When 50 µM of N-octanoyl dopamine was present, T-cell proliferation was still completely inhibited when 10 nM ciclosporin A or 1 nM tacrolimus. In view of the fact that 50 µM of N-octanoyl dopamine alone only partly inhibited T-cell proliferation at day 3 but not thereafter, this means that N-octanoyl dopamine and the calcineurin inhibitors act synergistically to inhibit T-cells. In fact, 50 µM of N-octanoyl dopamine can reduce the amount of calcineurin inhibitor required for complete T-cell suppression at least 1000 fold.

This demonstrates that the requirement for calcineurin inhibitors can be reduced by N-octanoyl dopamine without compromising immunosuppression. Since the nephrotoxicity of calcineurin inhibitors is directly related to their dose, a low dose ciclosporin A or tacrolimus treatment in combination with N-octanoyl dopamine will remain effective, but reduce the incidence of nephrotoxicity.

### Example 12

Cardiomyocytes were freshly isolated from new born rats and cultured for 3 days. Subsequently, they were stored for 8 hours at 4°C in the absence or presence of various concentrations of either dopamine or N-octanoyl dopamine, formulated according to Example 1. ATP levels and LDH release was measured after 8 hours of cold preservation and hereafter cardiomyocytes were re-warmed and the contraction rate per well and ATP regeneration were determined.

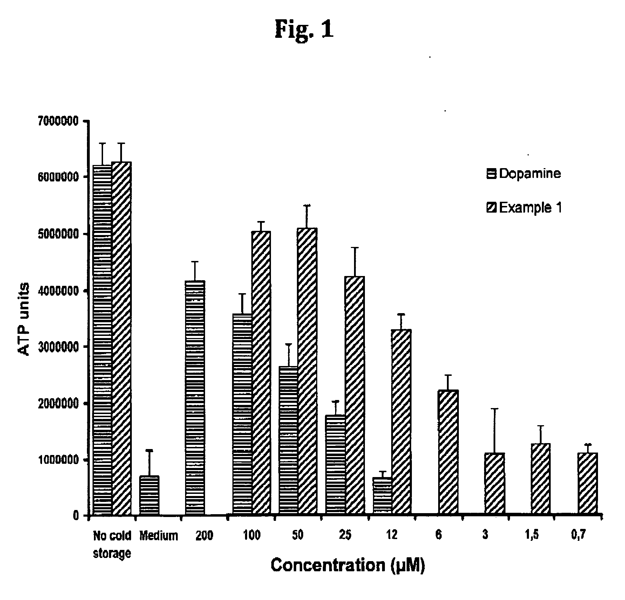

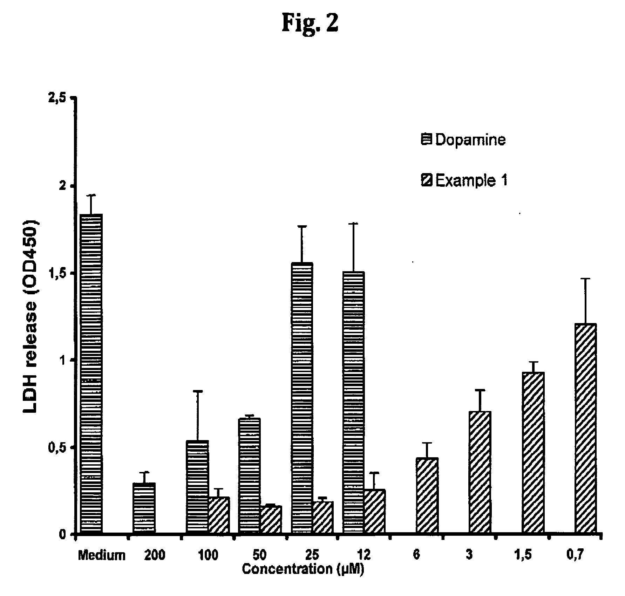

In result, ATP level measurement after 8 hours of cold storage revealed a dose-dependent mitigation of ATP loss in all treated cardiomyocytes compared with untreated cells (Figure 1). The effect of the composition of the invention was seen in much lower dosages than in the case of the dopamine composition. Also, regeneration of ATP after re-warming was much faster in treated cells compared to untreated cells. LDH-release after 8 hours of cold storage was inhibited in treated cells, but the N-octanoyl dopamine formulation was much more effective in low concentrations (Figure 2). After re-warming the cardiomyocytes, contraction rates revealed a significantly improved rate in treated cells.

The results demonstrate that N-octanoyl dopamine formulated according to the invention can prevent cold storage injury in cardiomyocytes, and is still protective in very low dosages, which is in contrast to conventional dopamine treatment. Since cold preservation and reperfusion injury are negative predictors for cardiac graft outcome, this suggest that the composition of the invention will be useful for donor preconditioning.

## Claims

1. A pharmaceutical composition comprising:
(a) an effective amount of N-octanoyl dopamine;
(b) a physiologically acceptable aqueous solvent; and
(c) a physiologically acceptable amphiphilic excipient;
wherein the N-octanoyl dopamine is present in a molecularly or colloidally dispersed state.

2. The composition of claim 1 in the form of a micellar solution or microemulsion.

3. The composition of claim 2, wherein the amphiphilic excipient is a nonionic surfactant.

4. The composition of claim 3, wherein the nonionic surfactant is a polysorbate.

5. The composition of claim 1 in the form of a liposomal dispersion.

6. The composition of claim 5, wherein the amphiphilic excipient is a vesicle-forming phospholipid.

7. The composition of any previous claim, having a pH of not higher than about 7.

8. The composition of any previous claim, further comprising an acid and/or an antioxidant

9. The composition of any of claims 1 to 8 for use as a medicine or as a preparation for organ or tissue preservation.

10. The composition of any of claims 1 to 8 for a use according to claim 9, wherein the composition is administered to an organ or tissue donor.

11. The composition of any of claims 1 to 8 for a use according to claim 9, wherein the composition is administered to an organ or tissue transplant recipient

12. The composition of any of claims 1 to 8 for a use according to claim 11, wherein the composition is administered parenterally.

13. A non-aqueous pharmaceutical composition comprising an effective amount of N-octanoyl dopamine and a physiologically acceptable amphiphilic excipient, being adapted to yield a composition according to any of claims 1 to 8 upon mixing with a physiologically acceptable aqueous solvent.

14. N-octanoyl dopamine for use in the prevention of organ or tissue transplant rejection in transplant recipients co-treated with a calcineurin inhibitors.

15. N-octanoyl dopamine for use according to claim 14, wherein the dose of the calcineurin inhibitor is lower than its effective dose in the absence of N-octanoyl dopamine co-treatrnent
